# EUROPEAN PATENT APPLICATION

(11) **EP 2 457 538 A1**
(43) Date of publication of application: **30.05.2012**
(21) Application number: 10802084.3
(22) Date of filing: 22.07.2010
(51) Int. Cl.: A61F 2/28

(54) **ARTIFICIAL BONE CONSTRUCTING UNIT AND ARTIFICIAL BONE CONSTRUCTING SYSTEM**

(30) Priority: 22.07.2009 JP 2009171665
(71) Applicant: NEXT21 K.K., Bunkyo-ku Tokyo 113-0033 (JP); The University of Tokyo, Bunkyo-Ku Tokyo 113-8654 (JP); Tama-Tlo, Tokyo 192-0083 (JP)
(72) Inventor: SUZUKI, Shigeki, Tokyo 113-0033 (JP); TEI, Yuichi, Tokyo 113-8654 (JP); SASAKI, Nobuo, Tokyo 113-8654 (JP); HE, Jianmei, Tokyo 163-8677 (JP)
(74) Representative: Ellis, Katherine Elizabeth Sleigh
(86) International application number: PCT/JP2010/004690
(87) International publication number: WO 2011/010463

(57) **Abstract**

Provided are an artificial bone constructing unit which is capable of being built into a free shape according to build-up design, and effectively guiding bone replacement, and an artificial bone system. Specifically disclosed are an artificial bone constructing unit and an artificial bone constructing system, wherein in principal, the artificial bone constructing unit which constructs an artificial bone is so made into a block shape that when blocks are assembled, continuous holes through a plurality of blocks are formed.

## Description

### Technical Field

The present invention relates to an artificial bone constituent unit capable of being assembled into a shape appropriate for a living body and effectively guiding bone replacement and an artificial bone constituent system.

### Background Art

Japanese Patent Application Laid-Open (JP-A) No. 11-19104 (Patent Document 1) discloses an artificial bone prosthesis. The artificial bone prosthesis has a guide groove for splitting and can be divided into an appropriate size by being split along the guide groove. Though the artificial bone prosthesis can be changed in shape to a certain extent, the flexibility of such a change is limited. Thus, the artificial bone prosthesis cannot build an artificial bone in an appropriate shape.

### Prior Art Document

### Patent Document

Patent Document 1: Japanese Patent Application Laid-Open (JP-A) No. 11-19104

### Summary of Invention

### Technical Problem

Therefore, an object of the present invention is to provide an artificial bone constituent unit capable of being assembled into a shape appropriate for a living body by a buildup design and effectively guiding bone replacement and an artificial bone constituent system.

### Solution to Problem

The present invention is basically based on findings that the above problem can effectively be solved by blocking artificial bone constituent units forming an artificial bone.

A first aspect of the present invention relates to an artificial bone constituent unit 1. The artificial bone constituent unit 1 is used as an artificial bone 3 by assembling a plurality of units thereof into an intended shape. The artificial bone 3 is also called a bone prosthetic agent or a bone filler. The artificial bone constituent unit 1 has a body portion 7 including a first hole 5. The body portion 7 has connecting portions 9, 11. The connecting portions 9, 11 are used to connect the artificial bone constituent unit 1 to one or a plurality of the other artificial bone constituent units 13, 15. Because of the above configuration, the artificial bone 3 in a desired shape can be obtained by assembling the plurality of the artificial bone constituent units 1.

In a preferred mode of the first aspect, the first hole 5 communicates with holes 17, 19 in other artificial bone constituent units 13, 15 connected to the artificial bone constituent unit 1. Therefore, due to the presence of a continuous hole throughout a plurality of blocks, it becomes easier for body tissues including blood vessel to enter an artificial bone. Accordingly, the artificial bone will effectively replace bone tissues.

In a preferred mode of the first aspect, the diameter of the first hole 5 is 0.1 mm or more and 1 cm or less. With the presence of a hole in this size, an optical amount of body tissues enters an artificial bone. Accordingly, the artificial bone will effectively replace bone tissues.

In a preferred mode of the first aspect, the body portion 7 further has a second hole 21 and a third hole 23. The second hole 21 is perpendicular to the first hole 5. The third hole 23 is perpendicular to the first hole 5 and the second hole 21. With the presence of such holes in various directions, body tissues effectively enter an artificial bone. Accordingly, the artificial bone will effectively replace bone tissues.

In a preferred mode of the first aspect, the body portion 7 is a block in a hexahedral structure. Because the artificial bone constituent unit 1 has such a shape, the unit can freely be designed into the shape of an artificial bone.

In a preferred mode of the first aspect, the body portion 7 is an isosceles trapezoidal column. Because the artificial bone constituent unit 1 has such a shape, the unit can freely be designed into the shape of an artificial bone.

In a preferred mode of the first aspect, the body portion 7 is a column and the cross section of the column is a portion of a ring. Because the artificial bone constituent unit 1 has such a shape, the unit can freely be designed into the shape of an artificial bone.

In a preferred mode of the first aspect, the body portion 7 is a polygonal column (for example, a regular triangular prism or a regular hexagonal prism). Because the artificial bone constituent unit 1 has such a shape, an artificial bone in various shapes can be designed by combining a plurality of artificial bone constituent units.

In a preferred mode of the first aspect, the body portion 7 is a regular triangular prism. Because the artificial bone constituent unit 1 has such a shape, an artificial bone suitable for a bone lacking region having a spherical structure such as the cranial bone can be designed.

In a preferred mode of the first aspect, the body portion 7 has a first groove 24 on the surface thereof. Because the artificial bone constituent unit 1 has the groove 24, an adhesive can easily be injected so that the connection of artificial bone constituent units can be made stronger.

In a preferred mode of the first aspect, the connecting portions 9, 11 further have second grooves 25, 26. When connected to another artificial bone constituent unit, the grooves 25, 26 fit in with grooves of the other artificial bone constituent unit to form a hole. Cells and blood vessel enter the formed hole. Accordingly, the connection between artificial bone constituent units becomes stronger.

In a preferred mode of the first aspect, the connecting portions 9, 11 are a convex portion 27 or a concave portion 28. The convex portion 27 or the concave portion 28 of the artificial bone constituent unit 1 fits into a concave portion 29 or a convex portion 31 of the other connected artificial bone constituent units 13, 15 respectively. The artificial bone constituent unit 1 in this mode is connected to the other artificial bone constituent units 13, 15 via the convex portion 27 or the concave portion 28.

In a preferred mode of the first aspect, the body portion 7 is an isosceles trapezoidal column. The artificial bone constituent unit 1 in this mode is provided with the connecting portions 9, 11 at least on isosceles side faces 33, 35 of the body portion 7. The artificial bone constituent unit 1 in this mode is connected to one or a plurality of the other artificial bone constituent units 13, 15 via the isosceles side faces.

A second aspect of the present invention relates to an artificial bone constituent system including the artificial bone constituent unit described above. This system is an artificial bone constituent system 40 including a plurality of artificial bone constituent units. The artificial bone constituent system 40 has at least a first artificial bone constituent unit group 41 and a second artificial bone constituent unit group 61. The first artificial bone constituent unit group 41 and the second artificial bone constituent unit group 61 have a plurality of first artificial bone constituent units 42 and a plurality of second artificial bone constituent units 62 respectively.

The first artificial bone constituent unit 42 has a body portion 44, first connecting portions 47, 48, and second connecting portions 51, 52. The body portion 44 is a portion that has a first hole 43 and a hexahedral structure. The first connecting portions 47, 48 are portions mounted on a left side face 45 and a right side face 46 of the body portion 44. The second connecting portions 51, 52 are portions mounted on a front 49 and a rear face 50 of the body portion 44. The first connecting portions 47, 48 are used to connect the first artificial bone constituent unit 42 to one or two artificial bone constituent units 53, 54 contained in the first artificial bone constituent unit group 41.

On the other hand, the second artificial bone constituent unit 62 has a body portion 64, first connecting portions 67, 68, and second connecting portions 71, 72. The body portion 64 is a portion that has a first hole 63 and a hexahedral structure. The first connecting portions 67, 68 are portions mounted on a left side face 65 and a right side face 66 of the body portion 64. The second connecting portions 71, 72 are portions mounted on a front 69 and a rear face 70 of the body portion 64. The first connecting portions 67, 68 are used to connect the second artificial bone constituent unit 62 to one or two artificial bone constituent units 73, 74 contained in the second artificial bone constituent unit group 61.

In the artificial bone constituent system, the second connecting portion 51 of the first artificial bone constituent unit 41 is connected to the second connecting portion 72 of the second artificial bone constituent unit 62. Accordingly, the first artificial bone constituent unit 42 and the second artificial bone constituent unit 62 are connected in the artificial bone constituent system.

In a preferred mode of the second aspect, if one or two first artificial bone constituent units are connected to the first artificial bone constituent unit group 41 via one of the first connecting portions 47, 48 of the first artificial bone constituent unit 42, the shape of a ring or a portion thereof is formed. Also in the artificial bone constituent system in this mode, if one or two second artificial bone constituent units are connected to the second artificial bone constituent unit group 61 via one of the first connecting portions 67, 68 of the second artificial bone constituent unit 62, the shape of a ring or a portion thereof is formed. Also in the artificial bone constituent system in this mode, the shape of the body portion 44 of the first artificial bone constituent unit 42 and the body portion 64 of the second artificial bone constituent unit 62 is a shape in which the shape of a ring or a portion thereof by the first artificial bone constituent unit group 41 is positioned on an outer circumference of the shape of a ring or a portion thereof by the second artificial bone constituent unit group 61. Accordingly, an artificial bone constituent system of the present invention can design an artificial bone by assembling artificial bone constituent unit like annual rings of a tree.

A third aspect of the present invention relates to an artificial bone constituent system 80 having a plurality of first artificial bone constituent units 82 and a plurality of second artificial bone constituent units 102. The first artificial bone constituent unit 82 has a body portion 88 and a connecting portion 92. The body portion 88 is a portion that has a first hole 84 and a second hole 86 and has the shape of a regular triangular prism. The connecting portion 92 is a portion mounted on a side face 90. The connecting portion 92 of the first artificial bone constituent unit 82 is used to connect to another first artificial bone constituent unit or the second artificial bone constituent unit 102.

The second artificial bone constituent unit 102 has a body portion 108 and a connecting portion 112. The body portion 108 is a portion that has a first hole 104 and a second hole 106 and has the shape of a regular triangular prism. The connecting portion 112 is a portion mounted on a side face 110 of the body portion 108. The connecting portion 112 is used to connect to the first artificial bone constituent unit 82 or another second artificial bone constituent unit.

The connecting portion 92 of the first artificial bone constituent unit 82 is connected to the connecting portion 112 of the second artificial bone constituent units 102, thereby connecting the first artificial bone constituent unit 82 and the second artificial bone constituent units 102. According to an artificial bone constituent system of the present invention obtained in this manner, an artificial bone of the desired size can be obtained by appropriately assembling the first artificial bone constituent unit 82 and the second artificial bone constituent units 102 fitting to the size of a bone lacking region. An artificial bone obtained from an artificial bone constituent system of the present invention can suitably be used as a portion of a spherical structure such as the cranial bone.

In a preferred mode of the third aspect of the present invention, an artificial bone constituent system further has a third artificial bone constituent unit 122. The third artificial bone constituent unit 122 has a body portion 128 and a connecting portion 132. The connecting portion 132 is a portion mounted on a side face 130 of the body portion 128. The connecting portion 132 is used to connect to the first artificial bone constituent unit 82, the second artificial bone constituent unit 102, or the other third artificial bone constituent unit. Accordingly, in an artificial bone constituent system 120, the first artificial bone constituent unit 82, the second artificial bone constituent unit 102, and the third artificial bone constituent unit 122 are connected. According to an artificial bone constituent system of the present invention obtained in this manner, an artificial bone of the desired size can be obtained by appropriately assembling the first artificial bone constituent unit 82, the second artificial bone constituent unit 102, and the third artificial bone constituent unit 122 fitting to the size of a bone lacking region. An artificial bone obtained from an artificial bone constituent system of the present invention can suitably be used as a portion of a spherical structure such as the cranial bone.

### Advantageous Effects of Invention

The present invention provides flexibility to the shape of an artificial bone by producing the artificial bone by assembling a plurality of artificial bone constituent units. Moreover, because a hole that is continuous throughout a plurality of blocks is present, body tissues including blood vessel enter the artificial bone. Accordingly, the artificial bone will effectively replace bone tissues.

### Brief Description of Drawings

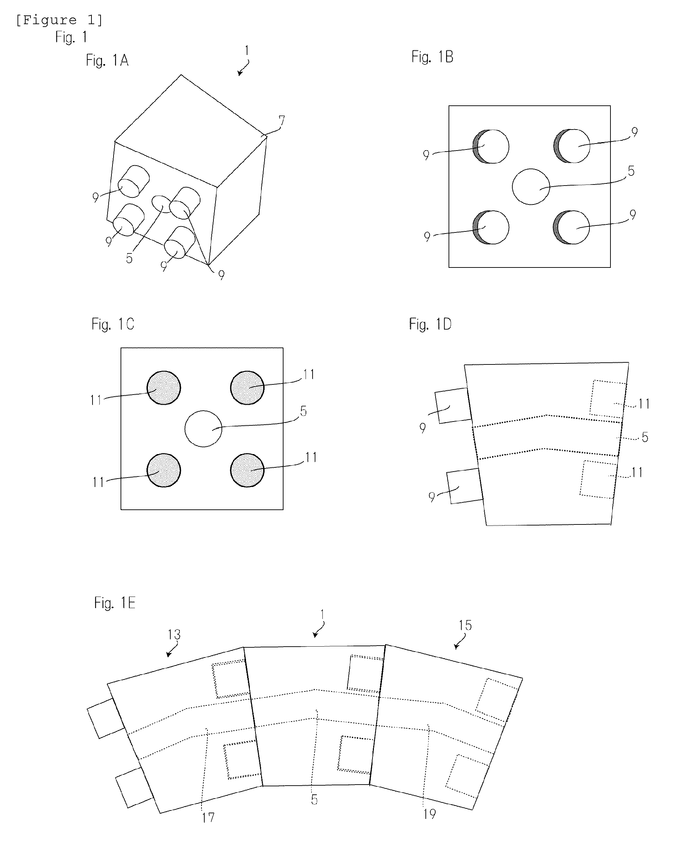

FIG. 1 is a diagram showing an example of an artificial bone constituent unit. FIG. 1A is a perspective view of the artificial bone constituent unit. FIG. 1B is a left side view of the artificial bone constituent unit. FIG. 1C is a right side view of the artificial bone constituent unit. FIG. 1D is a top view of the artificial bone constituent unit. FIG. 1E shows a top view of an artificial bone obtained by assembling three artificial bone constituent units.

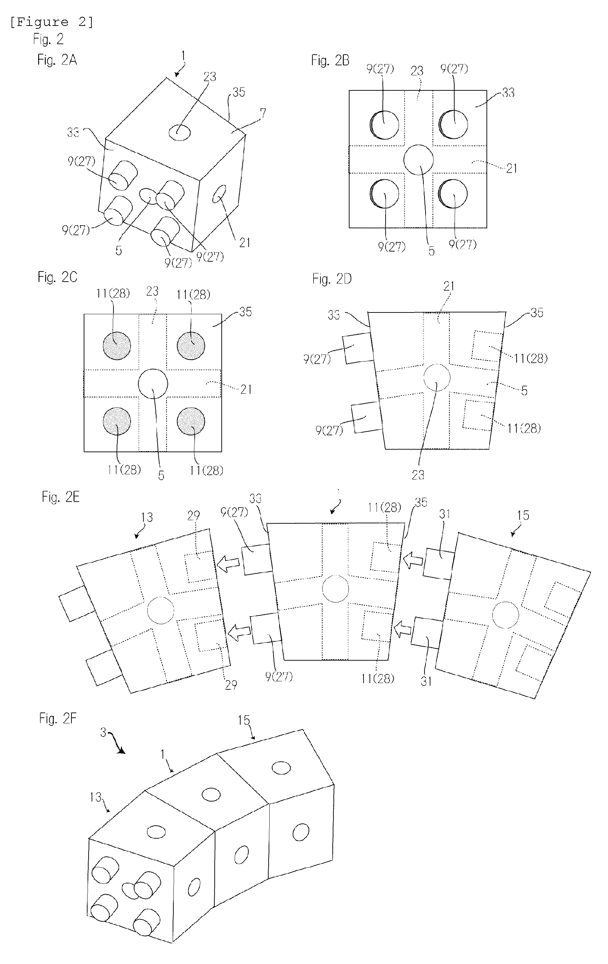

FIG. 2 is a diagram showing a different example from the above example of the artificial bone constituent unit. FIG. 2A is a perspective view of the artificial bone constituent unit. FIG. 2B is a left side view of the artificial bone constituent unit. FIG. 2C is a right side view of the artificial bone constituent unit. FIG. 2D is a top view of the artificial bone constituent unit. FIG. 2E shows a top view when three artificial bone constituent units are assembled. FIG. 2F shows a perspective view of the artificial bone when three artificial bone constituent units are assembled.

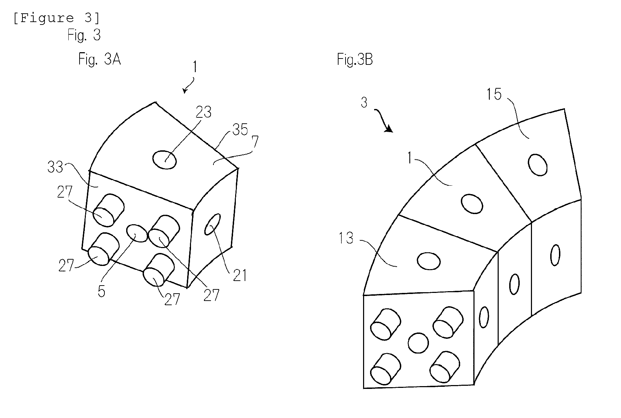

FIG. 3 is a diagram showing a still different example from the above examples of the artificial bone constituent unit. FIG. 3A is a perspective view of the artificial bone constituent unit. FIG. 3B shows a perspective view of the artificial bone when three artificial bone constituent units are assembled.

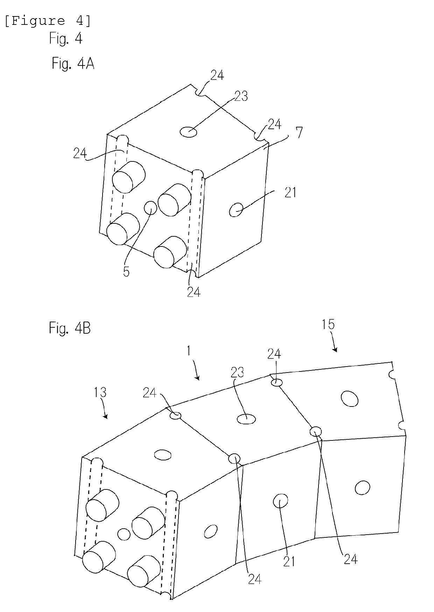

FIG. 4 is a diagram showing a still different example from the above examples of the artificial bone constituent unit. FIG. 4A is a perspective view of the artificial bone constituent unit. FIG. 4B shows a perspective view of the artificial bone when three artificial bone constituent units are assembled.

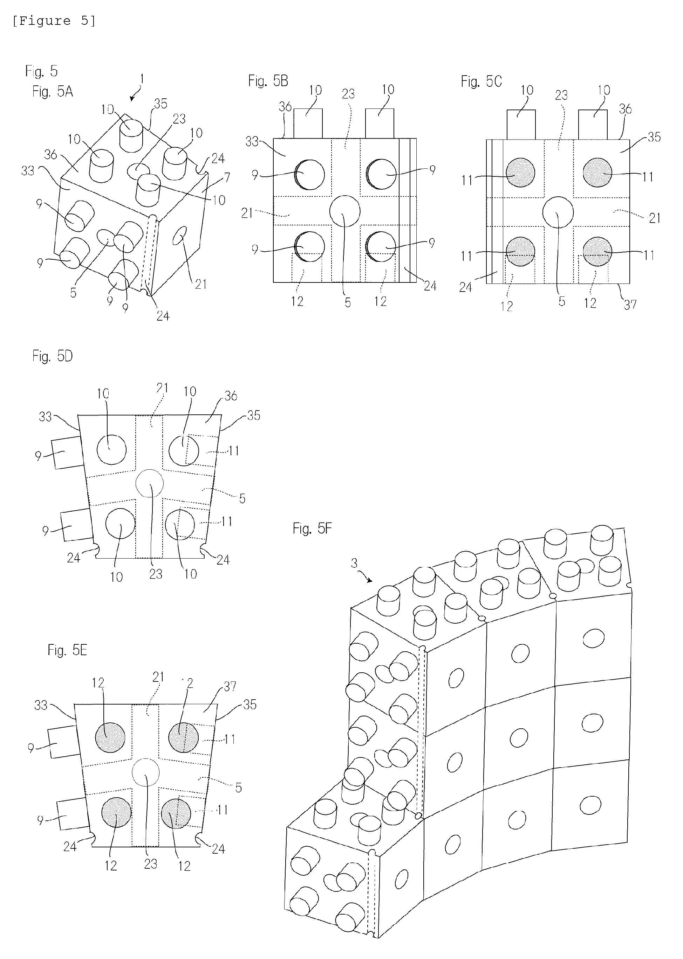

FIG. 5 is a diagram showing a still different example from the above examples of the artificial bone constituent unit. FIG. 5A is a perspective view of the artificial bone constituent unit. FIG. 5B is a left side view of the artificial bone constituent unit. FIG. 5C is a right side view of the artificial bone constituent unit. FIG. 5D is a top view of the artificial bone constituent unit. FIG. 5E is a bottom view of the artificial bone constituent unit. FIG. 5F shows a perspective view of the artificial bone when artificial bone constituent units are assembled.

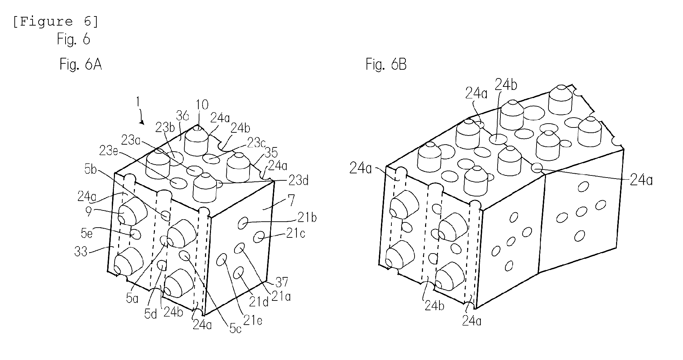

FIG. 6 is a diagram showing a still different example from the above examples of the artificial bone constituent unit. FIG. 6A is a perspective view of the artificial bone constituent unit. FIG. 6B is a perspective view of the artificial bone when artificial bone constituent units are assembled.

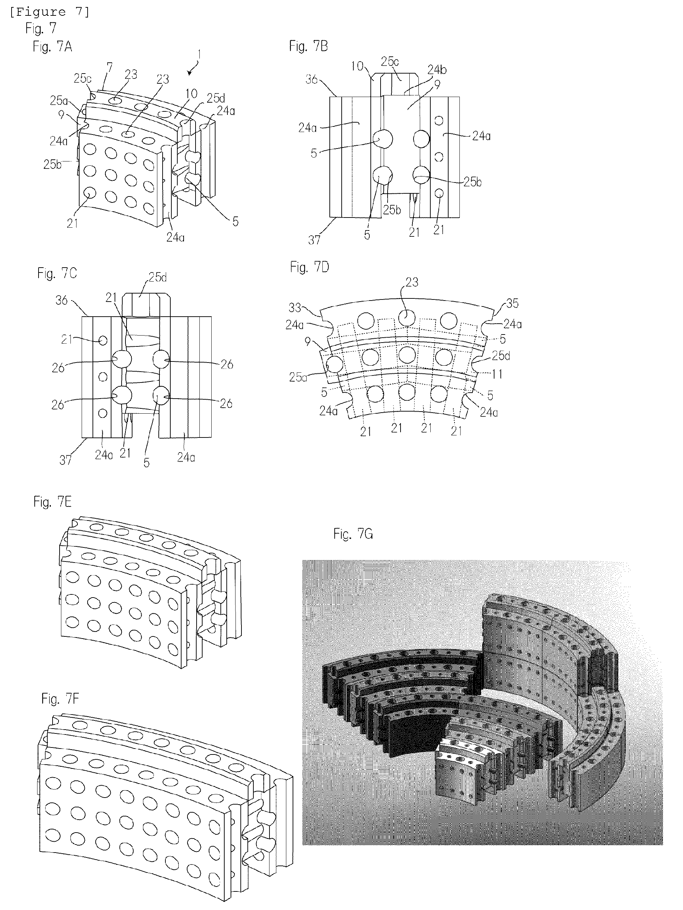

FIG. 7 is a diagram showing another artificial bone constituent unit from the above ones. FIG. 7A is a perspective view of the artificial bone constituent unit. FIG. 7B is a left side view of the artificial bone constituent unit. FIG. 7C is a right side view of the artificial bone constituent unit. FIG. 7D is a top view of the artificial bone constituent unit. FIGS. 7E and 7F are perspective views of artificial bone constituent units with different sizes. FIG. 7G is a perspective view of an artificial bone 3 when artificial bone constituent units with different sizes are assembled.

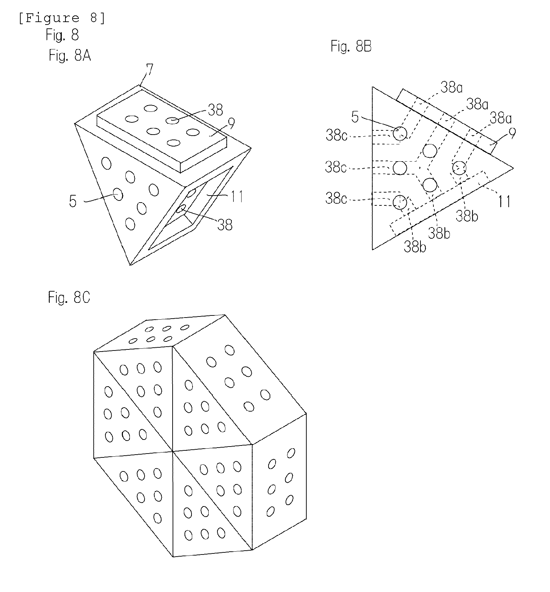

FIG. 8 is a diagram showing another artificial bone constituent unit from the above ones. FIG. 7A is a perspective view of the artificial bone constituent unit. FIG. 7B is a left side view of the artificial bone constituent unit. FIG. 7C is a right side view of the artificial bone constituent unit. FIG. 7D is a top view of the artificial bone constituent unit. FIGS. 7E and 7F are perspective views of artificial bone constituent units with different sizes. FIG. 7G is a perspective view of the artificial bone when artificial bone constituent units with different sizes are assembled.

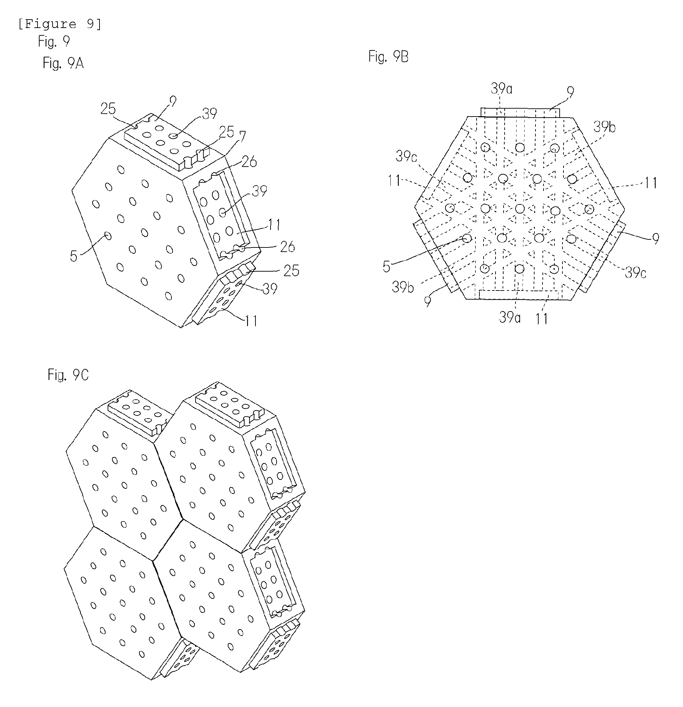

FIG. 9 is a diagram showing a still different example from the above examples of the artificial bone constituent unit. FIG. 9A is a perspective view of the artificial bone constituent unit. 9B is a front view of the artificial bone constituent unit. FIG. 9C shows a perspective view of the artificial bone when a plurality of artificial bone constituent units is assembled.

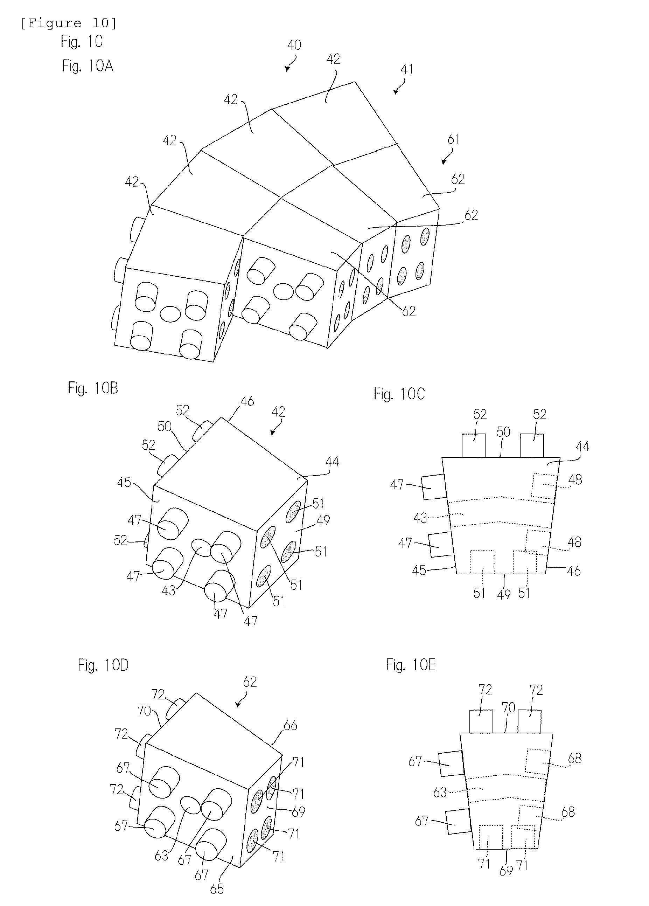

FIG. 10 is a diagram showing an example of an artificial bone constituent system. FIG. 10A is a perspective view of the artificial bone constituent system. FIG. 10B is a perspective view showing a first artificial bone constituent unit. FIG. 10C is a top view of the first artificial bone constituent unit. FIG. 10D is a perspective view showing a second artificial bone constituent unit. FIG. 10E is a top view of the second artificial bone constituent unit.

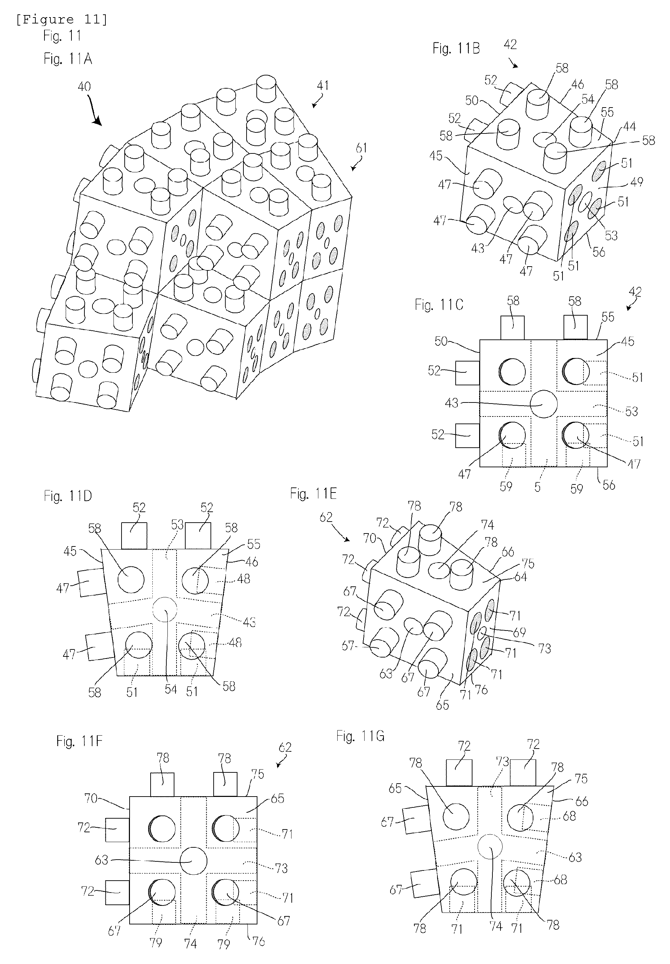

FIG. 11 is a diagram showing a different example from the above example of the artificial bone constituent system. FIG. 11A is a perspective view of the artificial bone constituent system. FIG. 11B is a perspective view showing the first artificial bone constituent unit. FIG. 11C is a left side view of the first artificial bone constituent unit. FIG. 11D is a top view of the first artificial bone constituent unit. FIG. 11E is a perspective view showing the second artificial bone constituent unit. FIG. 11F is a left side view of the second artificial bone constituent unit. FIG. 11G is a top view of the second artificial bone constituent unit.

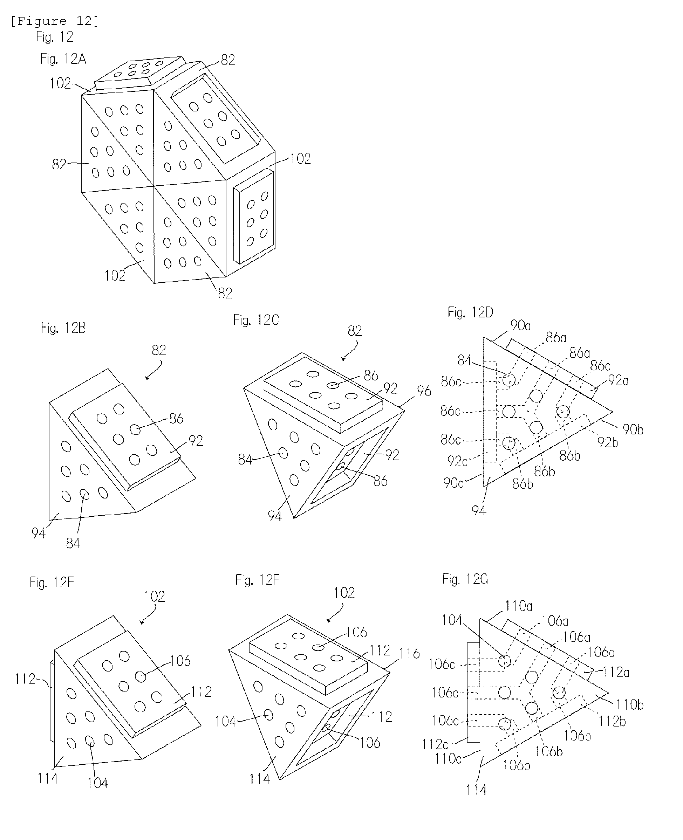

FIG. 12 is a diagram showing another artificial bone constituent system from the above ones. This artificial bone constituent system has a plurality of first artificial bone constituent units and a plurality of second artificial bone constituent units. FIG. 12A is a perspective view of the artificial bone constituent system. FIGS. 12B and 12C are perspective views of the first artificial bone constituent unit. FIG. 12D is a front view of the first artificial bone constituent unit. FIGS. 12E and 12F are perspective views of the second artificial bone constituent unit. FIG. 12G is a front view of the second artificial bone constituent unit.

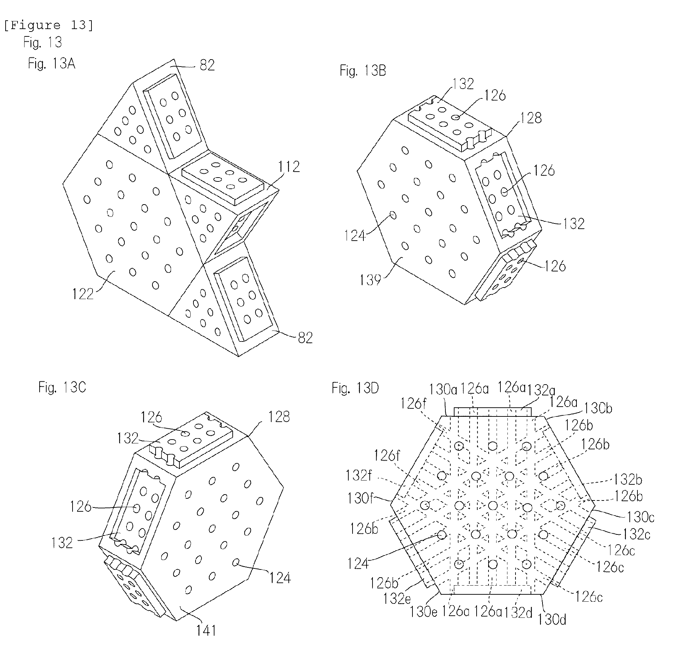

FIG. 13 is a diagram showing another artificial bone constituent system from the above ones. This artificial bone constituent system has, in addition to the first artificial bone constituent unit and the second artificial bone constituent unit described above, a third artificial bone constituent unit. FIG. 13A is a perspective view of the artificial bone constituent system. FIGS. 13B and 13C are perspective views of the third artificial bone constituent unit. FIG. 13D is a front view of the third artificial bone constituent unit.

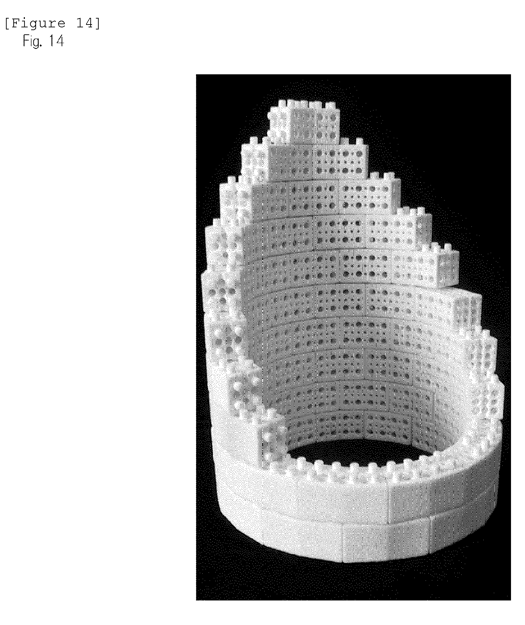

FIG. 14 is a photo, instead of a drawing, showing the artificial bone obtained by assembling artificial bone constituent units produced according to Example 1.

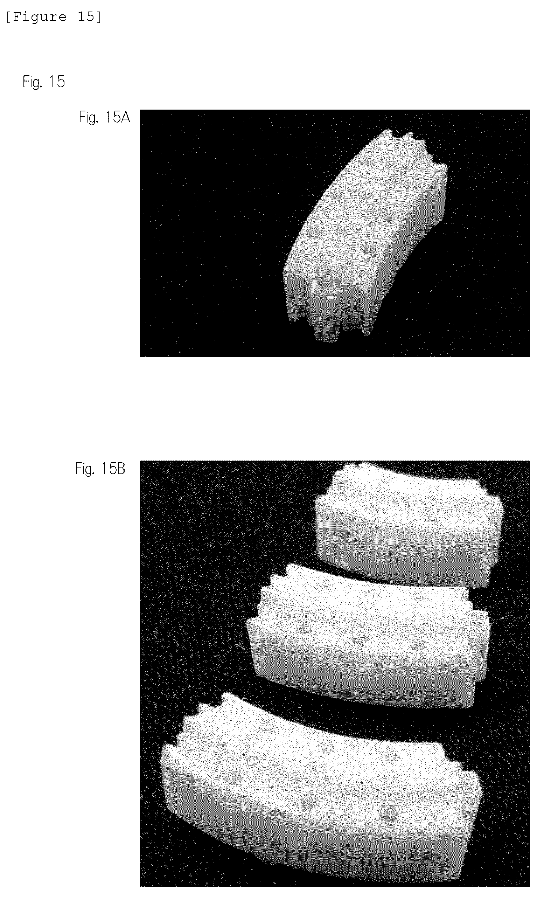

FIG. 15 shows photos, instead of drawings, showing the artificial bone constituent units produced according to Example 2. FIG. 15A shows the artificial bone constituent unit in the shape of a portion of a ring. FIG. 15B shows the artificial bone constituent unit forming a portion of the outer circumference of a ring and the artificial bone constituent unit forming a portion of a ring therewithin.

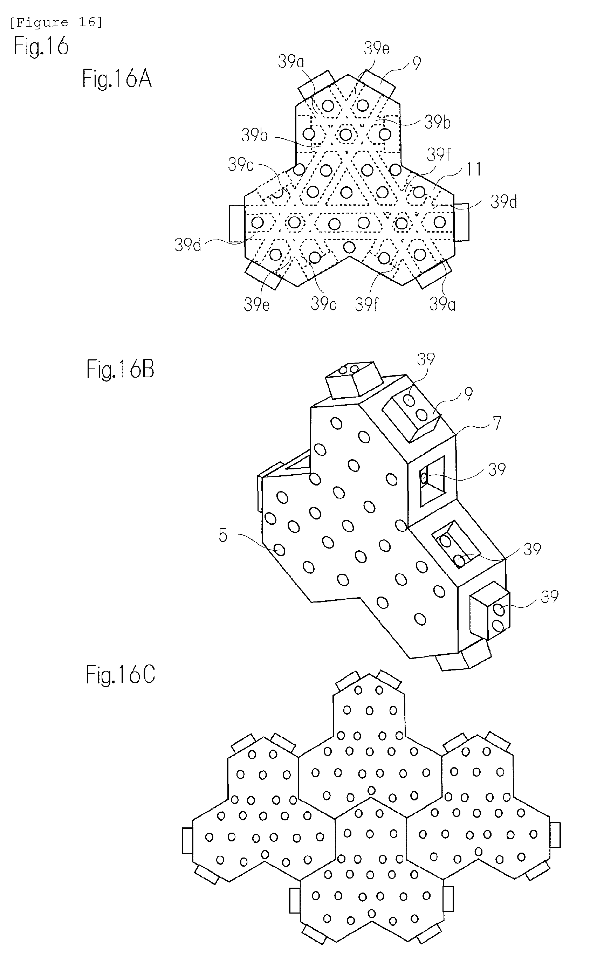

FIG. 16 is a diagram showing a still different example from the above examples of the artificial bone constituent unit. FIG. 16A is a front view of the artificial bone constituent unit. FIG. 16B is a perspective view of the artificial bone constituent unit. FIG. 16C shows a front view of the artificial bone when a plurality of artificial bone constituent units is assembled.

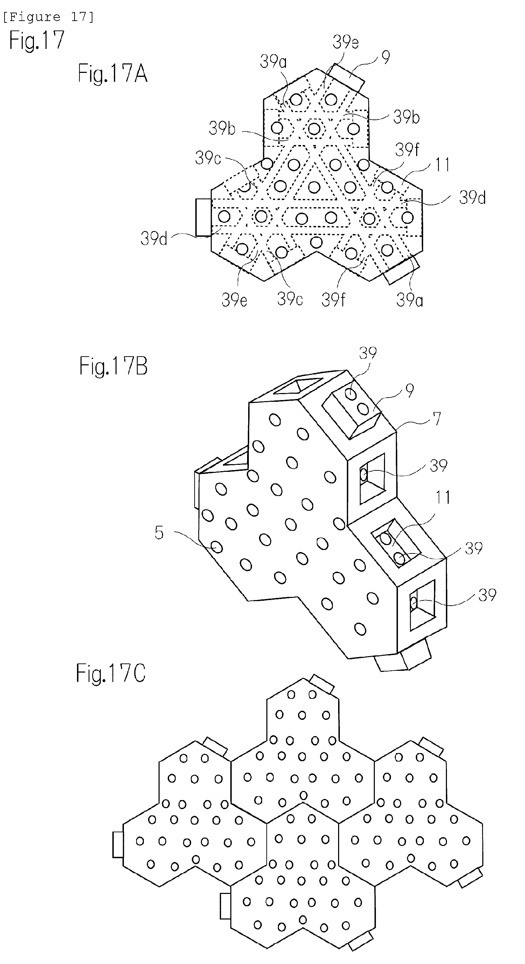

FIG. 17 is a diagram showing a still different example from the above examples of the artificial bone constituent unit. FIG. 17A is a front view of the artificial bone constituent unit. FIG. 17B is a perspective view of the artificial bone constituent unit. FIG. 17C shows a front view of the artificial bone when a plurality of artificial bone constituent units is assembled.

### Description of Embodiments

The embodiments to carry out the present invention will be described below based on drawings. FIG. 1 is a diagram showing an artificial bone constituent unit according to a first aspect of the present invention. FIG. 1A is a perspective view of an artificial bone constituent unit 1. FIG. 1B is a left side view of the artificial bone constituent unit 1. FIG. 1C is a right side view of the artificial bone constituent unit 1. FIG. 1D is a top view of the artificial bone constituent unit 1. FIG. 1E shows a top view of an artificial bone 3 obtained by assembling three artificial bone constituent units.

The artificial bone constituent unit 1 according to the first aspect of the present invention is used as an artificial bone by assembling a plurality thereof into an intended shape.

The artificial bone constituent unit contains artificial bone materials. An example of the artificial bone material used for an artificial bone constituent unit in the present invention is a calcium based material. Any calcium based material close to components of the bone may be used and is not specifically limited. Examples of the calcium based material include a calcium phosphate based material, a calcium carbonate based material, calcium lactate, and calcium gluconate. Among these calcium based materials, the calcium phosphate based material or the calcium carbonate based material is preferable. Examples of the calcium phosphate based material as material powder include hydroxyapatite, carbonate apatite, fluorine apatite, chlorine apatite,β-TCP, α-TCP, calcium metaphosphate, tetracalcium phosphate, octacalcium phosphate, calcium hydrogenphosphate, calcium dihydrogenphosphate, calcium pyrophosphate, or one or two of salts thereof or solvate thereof and, among these calcium phosphate based materials, α-TCP, β-TCP, or hydroxyapatite is preferable. Examples of the calcium carbonate based material include calcium carbonate and calcium hydrogencarbonate and, among these calcium carbonate based materials, calcium carbonate is preferable. The above calcium based materials may contain other compounds if necessary as long as the above compounds are main components. An artificial bone constituent unit produced from the above materials has a property of gradually replacing bone tissues in a living body. Therefore, an artificial bone constituent unit produced by using calcium based materials can suitably be used for treatment of bone lacking regions.

In the present invention, the ratio of calcium based materials in the artificial bone constituent unit is preferably 40 to 90 percents by weight when the weight of the artificial bone constituent unit is set to 100 percents by weight. In addition, the artificial bone constituent unit in the present invention preferably contains, in addition to the calcium based material, a finishing agent such as a dicarboxylic compound as well. The finishing agent can prevent a situation in which elution of calcium ions from the artificial bone constituent unit occurs. Elution of calcium ions from the artificial bone constituent unit could cause an inflammatory reaction and cytotoxicity. Therefore, the artificial bone constituent unit containing a finishing agent can prevent a situation in which body tissues in which artificial bone constituent units are set up are damaged by calcium ions eluted from the artificial bone constituent units. Further, the artificial bone constituent unit containing a finishing agent can prevent a situation in which a substance involved in growth of cells is adsorbed by the artificial bone constituent unit. Therefore, after the shape being provided to the artificial bone constituent unit in the present invention, the artificial bone constituent unit preferably contains a finishing agent such as trehalose. The artificial bone constituent unit in the present invention may contain a material needed for the formation of a bearer such as a binder of curable apatite as a secondary member. A person skilled in the art can use such a secondary member appropriately.

As shown in FIG. 1A, the artificial bone constituent unit 1 has a body portion 7. If the size of the body portion 7 is too small, it becomes difficult to assemble the artificial bone constituent units. If the size of the body portion 7 is too large, by contrast, it is difficult for an assembled artificial bone to match a desired shape. Thus, while the volume of the body portion 7 is cited as 0.1 cm³ or more and 40 cm³ or less, 0.2 cm³ or more and 30 cm³ or less is preferable, and 0.5 cm³ or more and 10 cm³ or less is particularly preferable.

In a preferred mode of the first aspect, as shown in FIG. 1A, the body portion 7 is a block in a hexahedral structure. Because the artificial bone constituent unit 1 has such a shape, the shape of an artificial bone can freely be designed. In the present invention, the hexahedral structure also contains a structure in which one or a plurality of edges or sides of the hexahedral structure is chamfered. By forming the body portion 7 as a block in a hexahedral structure for an artificial bone constituent unit in the present invention, an artificial bone can take a stable shape when assembled. In a preferred mode of the first aspect, the body portion 7 is an isosceles trapezoidal column. Because the artificial bone constituent unit 1 has such a shape, the shape of an artificial bone can freely be designed. In a preferred mode of the first aspect, the body portion 7 is a column and the cross section of the column is a portion of a ring. Because the artificial bone constituent unit 1 has such a shape, the shape of an artificial bone can freely be designed.

The body portion 7 has a first hole 5. As shown in FIGS. 1B and 1C, the first hole 5 is located on both left and right side faces. As shown in FIG. 1D, the first hole 5 preferably cuts through the body portion 7. In a preferred mode of the first aspect, the diameter of the first hole 5 is 0.1 mm or more and 1 cm or less. With the presence of a hole in this size, an optimal amount of body tissues enters an artificial bone. Accordingly, the artificial bone will effectively replace bone tissues. If the diameter of the hole is too small, it becomes difficult for blood vessel or cells to enter the hole. If the diameter of the hole is too large, the strength of an artificial bone constituent unit wanes. Thus, the diameter of the first hole is preferably 0.3 mm or more and 5 mm or less, particularly preferably 0.5 mm or more and 2 mm or less. The sectional shape of the hole may not be, as shown in FIG. 1B or 1C, circular. Examples of the shape of the hole other than the circular shape include an elliptic shape, polygonal shape, and star shape. If the artificial bone constituent unit has one of such shapes, blood vessel and cells can suitably enter deep into an assembled artificial bone. If blood vessel or cells enter the artificial bone, the time needed for the artificial bone to replace the bone in a living body can be reduced.

In a preferred mode of the first aspect, as shown in FIG. 1E, the first hole 5 communicates with holes 17, 19 present in other artificial bone constituent units 13, 15 connected to the artificial bone constituent unit 1. With the presence of a hole (communicating hole) communicating a plurality of blocks, it becomes easier for body tissues including blood vessel to enter an artificial bone. Accordingly, the artificial bone will effectively replace bone tissues.

Further, the body portion 7 has connecting portions 9, 11. The connecting portions 9, 11 are used to connect the artificial bone constituent unit 1 to one or a plurality of the other artificial bone constituent units 13, 15. With the above configuration, an artificial bone in a desired shape can be obtained by assembling a plurality of the artificial bone constituent units 1.

The connecting portions 9, 11 may have any shape that can connect the body portions 7. In the example shown in FIG. 1A, the connecting portion is formed of the convex portion 9 and the concave portion 11. The convex portion 9 and the concave portion 11 have shapes to fit in with each other.

FIG. 2 is a diagram showing a different example from the above example of the artificial bone constituent unit. FIG. 2A is a perspective view of the artificial bone constituent unit. FIG. 2B is a left side view of the artificial bone constituent unit. FIG. 2C is a right side view of the artificial bone constituent unit. FIG. 2D is a top view of the artificial bone constituent unit. FIG. 2E shows a top view when three artificial bone constituent units are assembled. FIG. 2F shows a perspective view of the artificial bone when three artificial bone constituent units are assembled.

In the example shown in FIG. 2, the body portion 7 further has a second hole 21 and a third hole 23. As shown in FIGS. 2A to 2D, the second hole 21 is perpendicular to the first hole 5. Also as shown in FIGS. 2A to 2D, the third hole 23 is perpendicular to the first hole 5 and the second hole 21. With the presence of such holes in various directions, body tissues effectively enter an artificial bone. Accordingly, the artificial bone will effectively replace bone tissues. In the example shown in FIGS. 2A to 2E, the second hole and the third hole are perpendicular to the first hole. However, the second hole and the third hole may not be perpendicular to the first hole.

If the ratio of volume occupied by the hole to the body portion is too small, it takes time before an artificial bone replaces the bone in a living body. On the other hand, if the ratio of volume occupied by the hole to the body portion is large, the strength of an artificial bone constituent unit wanes. Thus, if the volume of the body portion 7 including the hole is 100, while an example of the volume of the whole hole is 1 or more and 50 or less, 10 or more and 40 or less is preferable and, particularly preferably 10 or more and 30 or less. In the present invention, if the body portion 7 has a plurality of holes, the size of each hole may be the same or different.

In the example shown in FIG. 2, the body portion 7 is an isosceles trapezoidal column. In the artificial bone constituent unit 1 in this mode, the connecting portions 9, 11 are provided at least on isosceles side faces 33, 35 of the body portion 7. The artificial bone constituent unit 1 in this mode is connected to one or a plurality of the other artificial bone constituent units 13, 15 via the isosceles side faces. In this example, the connecting portions 9, 11 are the convex portion 27 or the concave portion 28. As shown in FIG. 2E, the convex portion 27 or the concave portion 28 of the artificial bone constituent unit 1 fits into a concave portion 29 or a convex portion 31 of the other connected artificial bone constituent units 13, 15 respectively. The artificial bone constituent unit 1 in this mode is connected to the other artificial bone constituent units 13, 15 via the convex portion 27 or the concave portion 28.

As shown in FIG. 2A, the body portion 7 may have a plurality of the convex portions 27 and a plurality of the concave portions 28. If the numbers of convex portions and concave portions are large, it becomes easier to fix an artificial bone constituent unit when connected to another artificial bone constituent unit, which is preferable. However, if there are too many convex portions and concave portions, it becomes almost impossible to provide holes of the desired size and the desired number. Therefore, one or more and eight or less is cited as the numbers of the convex portions and concave portions provided on one surface and the numbers thereof may be two or more and four or less. The convex portions and concave portions may also be provided on a plurality of surfaces.

Examples of the shape of the convex portion 27 include a cylinder, polygonal column, cone, polygonal cone, and tapering. The shape of a concave portion in the present invention may be any shape into which a convex portion is fitted. The artificial bone is unstable if the convex portion 27 is not sufficiently high. On the other hand, if the convex portion 28 is too high, it becomes more difficult to design an artificial bone. From the above points, the height of the convex portion 27 is preferably 0.1 mm or more and 5 mm or less, still preferably 0.2 mm or more and 3 mm or less, particularly preferably 0.4 mm or more and 2 mm or less.

FIG. 2F is a diagram showing a state in which three artificial bone constituent units are connected. As shown in FIG. 2F, the artificial bone is a curved artificial bone. That is, the artificial bone constituent unit in the present invention can reproduce a curved bone of living beings. Thus, an artificial bone fitting to an affected portion can be obtained by using an artificial bone constituent unit in the present invention.

FIG. 3 is a diagram showing a still different example from the above examples of the artificial bone constituent unit. FIG. 3A is a perspective view of the artificial bone constituent unit. FIG. 3B shows a perspective view of the artificial bone when three artificial bone constituent units are assembled.

As shown in FIG. 3A, the artificial bone constituent unit has the body portion 7 in a columnar shape and the cross section of the column is a portion of a ring. Because the artificial bone constituent unit 1 has such a shape, the shape of an artificial bone can freely be designed. Further, as shown in FIG. 3B, if the artificial bone 3 is built using the artificial bone constituent unit 1, a smooth artificial bone is built so that an artificial bone of lower invasion can be provided.

FIG. 4 is a diagram showing a still different example from the above examples of the artificial bone constituent unit. FIG. 4A is a perspective view of the artificial bone constituent unit. FIG. 4B shows a perspective view of the artificial bone 3 when three artificial bone constituent units are assembled.

As shown in FIG. 4A, the artificial bone constituent unit has groove 24 on the surface of the body portion 7. Because the artificial bone constituent unit has the groove 24, an adhesive can easily be injected to strengthen the connection between the artificial bone constituent units.

The groove 24 provided on the surface of the body portion 7 can be used as an adhesive injection hole. With an adhesive injected into the groove 24, the body portion 7 can enhance adhesiveness between adjacent artificial bone constituent units. The strength of an assembled artificial bone is thereby increased. Therefore, an artificial bone obtained by assembling artificial bone constituent units in the present invention can suitably be used also in a bone site under a load in a living body.

As shown in FIG. 4A, the groove 24 is preferably provided on one of the left and right side faces or both. When artificial bone constituent units are assembled, the groove 24 is preferably a groove that is continuous from an upper edge to a lower edge of the body portion and is preferably provided in a fixed position of the left and right side faces so that the groove 24 is continuous. The number of the grooves 24 may be one or two per surface as shown in FIG. 4A or three or more. Examples of the sectional shape of the groove 24 include a semicircle and polygon. The size of the groove 24 in a semicircular shape is 0.1 mm or more and 5 mm or less in diameter.

As shown in FIG. 4B, the groove 24 is preferably provided in each of corresponding positions of the adjacent two artificial bone constituent units. In such a case, the two grooves fit in to function as an adhesive injection hole.

FIG. 5 is a diagram showing a still different example from the above examples of the artificial bone constituent unit. FIG. 5A is a perspective view of the artificial bone constituent unit 1. FIG. 5B is a left side view of the artificial bone constituent unit 1. FIG. 5C is a right side view of the artificial bone constituent unit 1. FIG. 5D is a top view of the artificial bone constituent unit. FIG. 5E is a bottom view of the artificial bone constituent unit. FIG. 5F shows a perspective view of the artificial bone 3 when artificial bone constituent units are assembled.

As shown in FIGS. 5A to 5E, the body portion 7 of the artificial bone constituent unit has, in addition to the connecting portions 9, 11 on the isosceles side faces 33, 35, connecting portions 10, 12 on a top surface 36 and a undersurface 37 of the body portion 7. Further, the body portion 7 of the artificial bone constituent unit has the groove 24 on the surface of the isosceles side faces 33, 35. The body portion 7 is connected to another or a plurality of other artificial bone constituent units via the isosceles side faces 33, 35, the top surface 36, or the undersurface 37.

FIG. 5F is a diagram showing an example of the artificial bone 3 after the artificial bone constituent units 1 being assembled. As shown in FIG. 3, when the artificial bone constituent units are assembled, the groove 24 of the artificial bone constituent unit 1 is fitted in with the groove of the adjacent artificial bone constituent unit to form an adhesive injection hole. Moreover, the adhesive injection hole is formed continuously over a plurality of artificial bone constituent units so that many artificial bone constituent units can be fixed by injecting an adhesive from the top position.

FIG. 6 is a diagram showing a still different example from the above examples of the artificial bone constituent unit. FIG. 6A is a perspective view of the artificial bone constituent unit 1. FIG. 6B is a perspective view of the artificial bone 3 when artificial bone constituent units are assembled.

As shown in FIG. 6A, the body portion 7 of the artificial bone constituent unit has a hexahedral structure including a plurality of holes 5a to 5e, 21a to 21e, 23a to 23e. The body portion 7 has the connecting portions 9, 11 on the isosceles side faces 33, 35 and the connecting portions 10, 12 on the top surface 36 and the undersurface 37 of the body portion 7. Further, the body portion 7 has grooves 24a, 24b on the surface of the isosceles side faces 33, 35. The holes 5a, 5c, 5e are perpendicular to the holes 21a, 21c, 21e. The holes 5a, 5b, 5d are perpendicular to the holes 23a, 23c, 23e. The holes 21a, 21b, 21d are perpendicular to the holes 23a, 23b, 23d. The holes 5b, 5c, 5d, 5e are also perpendicular to the holes 21b, 23d, 21d, 23b respectively. The holes 21c, 21e are also perpendicular to the holes 23c, 23e respectively. In this way, the holes are communicatively connected to other holes.

As shown in FIG. 6A, the artificial bone constituent unit has a plurality of types of the grooves 24a, 24b. Further, the groove 24b is cut through by a hole in the artificial bone constituent unit. FIG. 6B is a diagram showing a state when the artificial bone constituent units are assembled. In the example shown in FIG. 6B, the two adjacent grooves 24b form one adhesive injection hole. The adhesive injection hole intersects holes in the vertical direction. Thus, when an adhesive is injected from the adhesive injection hole, the adhesive extends not only in the vertical direction, but also in the horizontal direction. As a result, the adhesive extending in the horizontal direction plays a role of a wedge so that the artificial bone constituent units are further strengthened.

FIG. 7 is a diagram showing another artificial bone constituent unit from the above ones. FIG. 7A is a perspective view of the artificial bone constituent unit 1. FIG. 7B is a left side view of the artificial bone constituent unit 1. FIG. 7C is a right side view of the artificial bone constituent unit 1. FIG. 7D is a top view of the artificial bone constituent unit 1. FIGS. 7E and 7F are perspective views of artificial bone constituent units with different sizes. FIG. 7G is a perspective view of the artificial bone 3 when artificial bone constituent units with different sizes are assembled.

In the example shown in FIG. 7, the body portion 7 of the artificial bone constituent unit has a structure in which a plurality of the holes 5, 21, 23 is included. The body portion 7 of the artificial bone constituent unit is in a columnar shape and the cross section of the column is a portion of a ring. A protruding portion forming a portion of a ring is present on the top surface and has a shape matching the shape of a depression at the bottom. On the other hand, a protruding portion is also present on the left side face of the body portion and has a shape matching the shape of a depression on the right side face. The protruding portion and the depression form a connecting portion. Each of the holes 5, 21, 23 intersects one of the holes and communicatively connected to other holes. As shown in FIGS. 7A to 7D, the body portion 7 has the connecting portions 9, 11 on the side faces 33, 35 and the connecting portions 10, 12 on the top surface 36 and the undersurface 37 of the body portion 7. Further, the body portion 7 has the grooves 24a, 24b on the surface of the side faces 33, 35. The connecting portions 9, 11 have the grooves 25 (25a, 25b), 26 and the connecting portion 10 has grooves 25c, 25d. The groove 25a is on the side of an adhesive surface of the connecting portion 9 and the side face 33 and is a portion cutting through from the side of the top surface 36 to the side of the undersurface 37. The groove 25a forms a hole by being fitted in with the groove 24b of the body portion 7. The grooves 25c, 25d included in the connecting portion 10 are portions on the side face on the side of the side faces 33, 35 of the body portion 7. The groove 25c on the side of the side face 33 is communicatively connected to the groove 24b on the side face. When artificial bone constituent units are assembled, the groove 24b, the groove 25a, and the groove 25c form a hole by being fitted in with grooves included in the connection portions of other artificial bone constituent units. When artificial bone constituent units are assembled, the grooves 25b, 26 included in the connecting portions 9, 11 also form a hole by being fitted in with grooves included in the connection portions of other artificial bone constituent units. If an artificial bone obtained by assembling such artificial bone constituent units is implanted in a human body, cells and blood vessel enter the hole formed by the groove 24b, the groove 25a, and the groove 25c or the hole formed by the grooves 25b, 26. The cells and blood vessel that have entered as described above can increase connection strength between artificial bone constituent units. Further, connection strength between artificial bone constituent units is increased by providing unevenness (groove) to the surface of the artificial bone constituent unit. Therefore, an artificial bone obtained by assembling such artificial bone constituent units can suitably be used also in a bone site likely to be under a load in a living body.

FIGS. 7E and 7F show artificial bone constituent units with different sizes. FIG. 7G shows a perspective view when artificial bone constituent units with different sizes as shown in FIGS. 7A, 7E, and 7F are combined. As shown in FIG. 7G, the artificial bone is a curved artificial bone. Moreover, the artificial bone can be made to have various degrees of curvature of the artificial bone. Therefore, such artificial bone constituent units can be assembled into an artificial bone having a desired size or a desired curve (curved surface) by fitting to the size of an affected portion and the artificial bone can suitably be used for a bone lacking region.

FIG. 8 is a diagram showing a still different example from the above examples of the artificial bone constituent unit. FIG. 8A is a perspective view of the artificial bone constituent unit. FIG. 8B is a front view of the artificial bone constituent unit. FIG. 8C is a perspective view of the artificial bone when artificial bone constituent units are assembled.

As shown in FIGS. 8A and 8B, the artificial bone constituent unit has the body portion 7 in a regular triangular prism shape. The body portion 7 of the artificial bone constituent unit in this mode has the connection portions 9, 11 on at least two surfaces of the three side faces. The body portion 7 has the first hole 5 and second holes 38. The first hole 5 is a hole cut through from the front to the rear face of the body portion 7. The second holes 38 (38a to 38c) are having an opening on the side face of the body portion. As shown in FIG. 8B, the first hole 5 and the second holes 38 (38a to 38c) each intersect other holes. Each hole is communicatively connected to other holes. If, as shown in FIG. 8C, artificial bone constituent units in a regular triangular prism shape are used, an artificial bone fitted to an affected portion in which a portion of the spherical structure such as the cranial bone is lacking can be obtained.

FIG. 9 is a diagram showing a still different example from the above examples of the artificial bone constituent unit. FIG. 9A is a perspective view of the artificial bone constituent unit. 9B is a front view of the artificial bone constituent unit. FIG. 9C shows a perspective view of the artificial bone when a plurality of artificial bone constituent units is assembled.

As shown in FIGS. 9A and 9B, the artificial bone constituent unit has the body portion 7 in a regular hexagonal prism shape. The body portion 7 of the artificial bone constituent unit in this mode has the connecting portion 9 in a convex shape or the connecting portion 11 in a concave shape on each of the six side faces. The connecting portions 9, 11 have a groove 39. In addition, the body portion 7 has the first hole 5 and second holes 39. The first hole 5 is a hole cut through from the front to the rear face of the body portion 7. The second holes 39 (39a to 39c) are holes cut through from one side face to the side face opposite thereto. As shown in FIG. 9B, the first hole 5 and the second holes 39 (39a to 39c) each intersect other holes. Each hole is communicatively connected to other holes. A portion of the second holes 39 is communicatively connected to the groove 39. When connected to another artificial bone constituent unit, the groove 39 is fitted in with the grove of the other artificial bone constituent unit to form a hole. If, as shown in FIG. 9C, artificial bone constituent units in a regular hexagonal prism shape are used, an artificial bone fitted to an affected portion in which a portion of the spherical structure such as the cranial bone is lacking can be obtained.

FIG. 16 is a diagram showing a still different example from the above examples of the artificial bone constituent unit. FIG. 16A is a front view of the artificial bone constituent unit. FIG. 16B is a perspective view of the artificial bone constituent unit. FIG. 17C shows a front view of the artificial bone when a plurality of artificial bone constituent units is assembled.

As shown in FIGS. 16A and 16B, the body portion 7 of the artificial bone constituent unit is a dihexagonal prism in which three regular hexagonal prisms are integrally combined. The body portion 7 of the artificial bone constituent unit in this mode has the six connecting portions 9 in the convex shape and the six connecting portions 11 in the concave shape on each of 12 side faces. The connecting portion 9 in the convex shape is provided on all six protruding sides of the body portion 7 of the dihexagonal prism and the connecting portion 11 in the concave shape is provided on all six recessed sides of the body portion 7 of the dihexagonal prism. The connecting portions 9, 11 have the groove 39. In addition, the body portion 7 has the first hole 5 and second holes 39. The first hole 5 is a hole cut through from the front to the rear face of the body portion 7. The second holes 39 (39a to 39f) are holes cut through from one side face to the side face opposite thereto. The first hole 5 and the second holes 39 (39a to 39f) may each be formed so as to intersect other holes. Each hole is communicatively connected to other holes. A portion of the second holes 39 is communicatively connected to the groove 39. When connected to another artificial bone constituent unit, the groove 39 is fitted in with the groove of the other artificial bone constituent unit to form a hole. If, as shown in FIG. 16C, artificial bone constituent units in a dihexagonal prism shape in which three regular hexagonal prisms are integrally combined are used, an artificial bone fitted to an affected portion in which a portion of the spherical structure such as the cranial bone is lacking can be obtained.

FIG. 17 is a diagram showing a still different example from the above examples of the artificial bone constituent unit. The artificial bone constituent unit shown in FIG. 17 is a modification of the artificial bone constituent unit shown in FIG. 16. FIG. 17A is a front view of the artificial bone constituent unit. FIG. 17B is a perspective view of the artificial bone constituent unit. FIG. 17C shows a front view of the artificial bone when a plurality of artificial bone constituent units is assembled.

As shown in FIGS. 17A and 17B, the body portion 7 of the artificial bone constituent unit is a dihexagonal prism in which three regular hexagonal prisms are integrally combined. The body portion 7 of the artificial bone constituent unit in this mode has the three connecting portions 9 in the convex shape and the nine connecting portions 11 in the concave shape on each of 12 side faces. The connecting portion 9 in the convex shape is provided on three sides of six protruding sides of the body portion 7 of the dihexagonal prism and the connecting portion 11 in the concave shape is provided on the remaining three sides. Also, the connecting portion 11 in the concave shape is provided on all six recessed sides of the body portion 7 of the dihexagonal prism. The connecting portions 9, 11 have the groove 39. In addition, the body portion 7 has the first hole 5 and the second holes 39. The first hole 5 is a hole cut through from the front to the rear face of the body portion 7. The second holes 39 (39a to 39f) are holes cut through from one side face to the side face opposite thereto. The first hole 5 and the second holes 39 (39a to 39f) may each be formed so as to intersect other holes. Each hole is communicatively connected to other holes. A portion of the second holes 39 is communicatively connected to the groove 39. When connected to another artificial bone constituent unit, the groove 39 is fitted in with the groove of the other artificial bone constituent unit to form a hole. If, as shown in FIG. 17C, artificial bone constituent units in a dihexagonal prism shape in which three regular hexagonal prisms are integrally combined are used, an artificial bone fitted to an affected portion in which a portion of the spherical structure such as the cranial bone is lacking can be obtained.

Comparison of the artificial bone constituent unit shown in FIG. 16 and the artificial bone constituent unit shown in FIG. 17 shows that the former has more connection portions with other artificial bone constituent units. Thus, an artificial bone obtained by assembling the artificial bone constituent unit shown in FIG. 16 has properties of high strength and being less likely to be detached. On the other hand, comparison of the artificial bone constituent unit shown in FIG. 16 and the artificial bone constituent unit shown in FIG. 17 shows that the latter has less connection portions with other artificial bone constituent units. Thus, an artificial bone obtained by assembling the artificial bone constituent unit shown in FIG. 17 has properties of high flexibility and being easy to assemble. Therefore, the artificial bone constituent units shown in FIGS. 16 and 17 each have different properties and a suitable artificial bone constituent unit depending on properties of the bone, which is an affected portion, may be selected.

FIG. 10 is a diagram showing an artificial bone constituent system 40 in the present invention. The artificial bone constituent system 40 contains a plurality of artificial bone constituent units. The artificial bone constituent system 40 has at least a first artificial bone constituent unit group 41 and a second artificial bone constituent unit group 61. The first artificial bone constituent unit group 41 and the second artificial bone constituent unit group 61 have a plurality of first artificial bone constituent units 42 and a plurality of second artificial bone constituent units 62 respectively. FIG. 10A is a perspective view of the artificial bone constituent system 40. FIG. 10B is a perspective view showing the first artificial bone constituent unit 42. FIG. 10C is a top view of the first artificial bone constituent unit 42. FIG. 10D is a perspective view showing the second artificial bone constituent unit 62. FIG. 10E is a top view of the second artificial bone constituent unit 62.

As shown in FIGS. 10B and 10C, the first artificial bone constituent unit 42 has a body portion 44, first connecting portions 47, 48, and second connecting portions 51, 52. The body portion 44 is a portion that has a first hole 43 and a hexahedral structure. The first connecting portions 47, 48 are portions mounted on a left side face 45 and a right side face 46 of the body portion 44. The second connecting portions 51, 52 are portions mounted on the front 49 and the rear face 50 of the body portion 44. The first connecting portions 47, 48 are used to connect the first artificial bone constituent unit 42 to one or two first artificial bone constituent units 53, 54 contained in the first artificial bone constituent unit group 41.

On the other hand, as shown in FIGS. 10D and 10E, the second artificial bone constituent unit 62 has the body portion 64, first connecting portions 67, 68, and second connecting portions 71, 72. The body portion 64 is a portion that has a first hole 63 and a hexahedral structure. The first connecting portions 67, 68 are portions mounted on the left side face 65 and the right side face 66 of the body portion 64. The second connecting portions 71, 72 are portions mounted on a front 69 and a rear face 70 of the body portion 64. The first connecting portions 67, 68 are used to connect the second artificial bone constituent unit 62 to the one or two second artificial bone constituent units 73, 74 contained in the second artificial bone constituent unit group 61.

In the artificial bone constituent system 40, the second connecting portion 51 of the first artificial bone constituent unit 41 is connected to the second connecting portion 72 of the second artificial bone constituent unit 62. Accordingly, the first artificial bone constituent unit 42 and the second artificial bone constituent unit 62 are connected in the artificial bone constituent system 40 of the present invention. As shown in FIG. 10A, an artificial bone constituent system in the present invention contains artificial bone constituent units of different sizes and therefore, an artificial bone fitted to the shape of a bone of living beings can be assembled.

FIG. 11 is a diagram showing a different example from the above example of the artificial bone constituent system 40. The artificial bone constituent system 40 contains a plurality of artificial bone constituent units. The artificial bone constituent system 40 has at least the first artificial bone constituent unit group 41 and the second artificial bone constituent unit group 61. The first artificial bone constituent unit group 41 and the second artificial bone constituent unit group 61 have the plurality of first artificial bone constituent units 42 and the plurality of second artificial bone constituent units 62 respectively. FIG. 11A is a perspective view of the artificial bone constituent system 40. FIG. 11B is a perspective view showing the first artificial bone constituent unit 42. FIG. 11C is a left side view of the first artificial bone constituent unit 42. FIG. 11D is a top view of the first artificial bone constituent unit 42. FIG. 11E is a perspective view showing the second artificial bone constituent unit 62. FIG. 11F is a left side view of the second artificial bone constituent unit 62. FIG. 11G is a top view of the second artificial bone constituent unit 62.

As shown in FIGS. 11B to 11D, the first artificial bone constituent unit 42 has the body portion 44 including the first connecting portions 47, 48, the second connecting portions 51, 52, and third connecting portions 58, 59. The body portion 44 has the first hole 43, a second hole 53, and a third hole 54 and has a hexahedral structure. The first connecting portions 47, 48 are portions mounted on the left side face 45 and the right side face 46 of the body portion 44. The second connecting portions 51, 52 are portions mounted on the front 49 and the rear face 50 of the body portion 44. The third connecting portions 58, 59 are portions mounted on a top surface 55 and an undersurface 56 of the body portion. The first connecting portions 47, 48 are used to connect the first artificial bone constituent unit 42 to the one or two artificial bone constituent units 53, 54 contained in the first artificial bone constituent unit group 41.

On the other hand, as shown in FIGS. 11E to 11G, the second artificial bone constituent unit 62 has the first connecting portions 67, 68, the second connecting portions 71, 72, and third connecting portions 78, 79. The body portion 64 has the first hole 63, a second hole 73, and a third hole 74 and has a hexahedral structure. The first connecting portions 67, 68 are portions mounted on the left side face 65 and the right side face 66 of the body portion 64. The second connecting portions 71, 72 are portions mounted on the front 69 and the rear face 70 of the body portion 64. The third connecting portions 78, 79 are portions mounted on a top surface 75 and an undersurface 76 of the body portion 64. The first connecting portions 67, 68 are used to connect the second artificial bone constituent unit 62 to the one or two artificial bone constituent units 73, 74 contained in the second artificial bone constituent unit group 61.

If one or two first artificial bone constituent units are connected to the first artificial bone constituent unit group 41 via one of the first connecting portions 47, 48 of the first artificial bone constituent unit 42, the shape of a ring or a portion thereof may be formed. If one or two second artificial bone constituent units are connected to the second artificial bone constituent unit group 61 via one of the first connecting portions 67, 68 of the second artificial bone constituent unit 62, the shape of a ring or a portion thereof may be formed. Incidentally, the shape of the body portion 44 of the first artificial bone constituent unit 42 and the body portion 64 of the second artificial bone constituent unit 62 is preferably a shape in which the shape of a ring or a portion thereof by the first artificial bone constituent unit group 41 is positioned on an outer circumference of the shape of a ring or a portion thereof by the second artificial bone constituent unit group 61.

FIG. 12 is a diagram showing an artificial bone constituent system 80 different from the above ones. The artificial bone constituent system 80 has a plurality of first artificial bone constituent units 82 and a plurality of second artificial bone constituent units 102. FIG. 12A is a perspective view of the artificial bone constituent system 80. FIGS. 12B and 12C are perspective views of the first artificial bone constituent unit 80. FIG. 12D is a front view of the first artificial bone constituent unit 80. FIGS. 12E and 12F are perspective views of the second artificial bone constituent unit 102. FIG. 12G is a front view of the second artificial bone constituent unit 102.

As shown in FIGS. 12B to 12D, a body portion 88 of the first artificial bone constituent unit 82 is a regular triangular prism having a first hole 84 and a second hole 86. The body portion 88 has the first hole 84 and the second holes 86. The first hole 84 is a hole cutting through from a front 94 to a rear face 96 of the body portion 88. The second holes 86 (86a to 86c) are holes having an opening on side faces 90 (90a to 90c) of the body portion 88. As shown in FIG. 12D, the first hole 84 and the second holes 86 (86a to 86c) each intersect other holes and are communicatively connected to other holes. The body portion 147 further has connecting portions 92 (92a to 92c) on the side faces 90 (90a to 90c) respectively. As shown in FIG. 12B to 12D, the connecting portion 92a has a convex shape and the connecting portions 92b, 92c have a concave shape. The convex shape and the concave shape of the connecting portions are shapes that fit together. The connecting portion 92a of the first artificial bone constituent unit 82 is connected to the connecting portion in the concave shape of another first artificial bone constituent unit or the second artificial bone constituent unit 102. In contrast, the connecting portions 92b, 92c of the first artificial bone constituent unit 82 are connected to the connecting portion in the convex shape of another first artificial bone constituent unit or the second artificial bone constituent unit 102.

On the other hand, as shown in FIGS. 12E to 12G, a body portion 108 of the second artificial bone constituent unit 102 is a regular triangular prism having a first hole 104 and a second hole 106. The body portion 108 has the first hole 104 and the second holes 106. The first hole 104 is a hole cutting through from a front 114 to a rear face 116 of the body portion 108. The second holes 106 (106a to 106c) are holes having an opening on side faces 110 (110a to 110c) of the body portion 108. As shown in FIG. 12G, the first hole 104 and the second holes 106 (106a to 106c) each intersect other holes and are communicatively connected to other holes. The body portion 108 further has connecting portions 112 (112a to 112c) on the side faces 110 (110a to 110c) respectively. As shown in FIG. 12E to 12G, the connecting portions 112a, 112c have a convex shape and the connecting portion 112b has a concave shape. The connecting portions 112a, 112c of the second artificial bone constituent unit 102 are connected to the connecting portion in the concave shape of the first artificial bone constituent unit 82 or another second artificial bone constituent unit. In contrast, the connecting portion 112b of the second artificial bone constituent unit 102 is connected to the connecting portion in the convex shape of the first artificial bone constituent unit 82 or another second artificial bone constituent unit.

In the artificial bone constituent system 80, as described above, the first artificial bone constituent unit 82 is connected to the second artificial bone constituent unit 102. As shown in FIG. 12A, an artificial bone constituent system according to the present invention can build an artificial bone as part of a spherical structure by combining a plurality of the first artificial bone constituent units 82 and a plurality of the second artificial bone constituent units 102. Therefore, the artificial bone constituent system according to the present invention can suitably be used for a deficiency of a region having the spherical structure such as the cranial bone.

FIG. 13 is a diagram showing an artificial bone constituent system 120 different from the above ones. This artificial bone constituent system 120 has, in addition to the first artificial bone constituent unit 82 and the second artificial bone constituent unit 102 described above, a third artificial bone constituent unit 122. FIG. 13A is a perspective view of the artificial bone constituent system 120. FIGS. 13B and 13C are perspective views of the third artificial bone constituent unit 122. FIG. 13D is a front view of the third artificial bone constituent unit 122.

As shown in FIG. 13B to 13D, the body portion 128 of the artificial bone constituent unit 122 is a regular hexagonal prism having a first hole 124 and second holes 126. The body portion 128 may have the first hole 124 and the second hole 126. The first hole 124 is a hole cut through from a front 139 to a rear face 141. The second holes 126 (126a to 126c) cut through from side faces 130a, 130b, 130c to side faces 130d, 130e, 130f opposite thereto respectively. As shown in FIG. 13D, the first hole 124 and the second holes 126 (126a to 126c) each intersect other holes and are communicatively connected to other holes. The body portion 128 further has connecting portions 132 (132a to 132f) on the side faces 130 (130a to 130f) respectively. As shown in FIG. 13B to 13D, the connecting portions 132a, 132c, 132e are in a convex shape and the connecting portions 132b, 132d, 132f are in a concave shape.

In the artificial bone constituent system 120 according to the present invention, the connecting portions 132a, 132c, 132e of the third artificial bone constituent unit 122 are connected to connecting portions in the concave shape of the first artificial bone constituent unit 84, the second artificial bone constituent unit 102, or another third artificial bone constituent unit. In contrast, the connecting portions 132b, 132d, 132f of the third artificial bone constituent unit 122 are connected to connecting portions in the convex shape of the first artificial bone constituent unit 84, the second artificial bone constituent unit 102, or another third artificial bone constituent unit. Also in the artificial bone constituent system 120 according to the present invention, the first artificial bone constituent unit 84 and the second artificial bone constituent unit 102 may be connected via the respective connecting portions. In this manner, the first artificial bone constituent unit 84, the second artificial bone constituent unit 102, and the third artificial bone constituent unit 122 are connected in the artificial bone constituent system 120.

As shown in FIG. 13A, an artificial bone constituent system according to the present invention can assemble an artificial bone as a portion of the spherical structure by combining a plurality of the first artificial bone constituent units 82, a plurality of the second artificial bone constituent units 102, and a plurality of the third artificial bone constituent units 122. Therefore, the artificial bone constituent system according to the present invention can suitably be used for a deficiency of a region having the spherical structure such as the cranial bone.

An artificial bone constituent unit according to the present invention can be produced by using publicly known artificial bone materials and publicly known production methods. An example of the production method of the artificial bone constituent unit according to the present invention is injection molding. An example of the production method of the artificial bone constituent unit will briefly be described below. The example of the production method is a method disclosed by WO 2007/094134. This production method of the artificial bone constituent unit includes a kneading process, a molding process, a de-binder (degreasing) process, and a sintering process. The kneading process is a process to knead raw materials including a calcium based material and materials including a binder. The molding process is a process to obtain a molded body having a predetermined shape by using a kneaded material obtained in the kneading process through injection molding using an injection molding machine having a die. The de-binder (degreasing) process is a process to obtain a degreased body by removing a binder contained in the molded body obtained in the molding process. The sintering process is a process to obtain a sintered body by heating the degreased body after the de-binder process for sintering. In the present invention, a cleaning process may further be included after the sintering process. Any person skilled in the art can perform a publicly known post-processing process to appropriately perform post-processing of the molded body.

As another production method, a curing agent solution is added to a curable artificial bone material having calcium phosphate or calcium carbonate as a main component by using a die cutting production method and the curable artificial bone material is kneaded and after the curable artificial bone material is cured, the artificial bone material is pulled out of the molding die.

An example of the artificial bone material used for an artificial bone constituent unit according to the present invention is a calcium based material. Any calcium based material close to components of the bone may be used and is not specifically limited. Examples of the calcium based material include a calcium phosphate based material, a calcium carbonate based material, calcium lactate, and calcium gluconate. Among these calcium based materials, the calcium phosphate based material or the calcium carbonate based material is preferable. Examples of the calcium phosphate based material as material powder include hydroxyapatite, carbonate apatite, fluorine apatite, chlorine apatite, β-TCP, α-TCP, calcium metaphosphate, tetracalcium phosphate, octacalcium phosphate, calcium hydrogenphosphate, calcium dihydrogenphosphate, calcium pyrophosphate, or one or two of salts thereof or solvate thereof and, among these calcium phosphate based materials, α-TCP, β-TCP, or hydroxyapatite is preferable. Examples of the calcium carbonate based material include calcium carbonate and calcium hydrogencarbonate and, among these calcium carbonate based materials, calcium carbonate is preferable. The above calcium based materials may contain other compounds if necessary as long as the above compounds are main components. An artificial bone constituent unit produced from the above materials has a property of gradually replacing bone tissues in a living body. Therefore, an artificial bone constituent unit produced by using calcium based materials can suitably be used for treatment of bone lacking regions.

Using an artificial bone constituent unit according to the present invention, an artificial bone is formed by assembling a plurality of such units. Then, the artificial bone is implanted in a patient who needs such an artificial bone. Thus, the artificial bone constituent unit according to the present invention is effective in treatment of patients who need such an artificial bone. That is, the present invention also provides a treating method of humans and mammals other than humans using the above artificial bone constituent unit according to the present invention.

### Example 1

Artificial bone constituent units are actually produced and assembled. The body portion is a hexagon the size of one side of which is about 5 mm. The height of a connecting portion is set to about 1 mm. The artificial bone constituent units are produced by the production method disclosed by WO 2007/094134.

### (1) Kneading process

α-TCP (manufactured by Taihei Chemical Industrial Co., Ltd., grain size: 10 µm) is used as material powder. A binder is formulated so that the percentage thereof by weight is 24 when the percentage of the material powder by weight is set to 100. As the binders, ethylene-vinyl acetate copolymer, poly(t-butyl methacrylate), paraffin wax, dibutyl phthalate, and stearic acid are used in a blending ratio of 30:30:30:5:5 by weight. A pressure kneader of 300 cc is heated to 150°C, the binders are input in descending order of melting point and after all binders are input, the binders are kneaded for 60 min and then cooled. The obtained kneaded material is ground by a pot mill made of ceramics for use as a material of molding (compound or pellet).

### (2) Molding process

The die is produced according to a CAD image of a bone prosthetic agent after the image thereof is formed by using CAD. A horizontal injection molding machine whose mold clamping force is 12 tons is used. The initial setting of the injection pressure is set to 12 GPa. The temperature of the cylinder of the molding machine is set to 130°C and the temperature of the die to 20°C.

### (3) De-binder process

An atmospheric degreasing furnace is heated to the highest temperature in an atmospheric air (for example, in the range of 450 to 550°C) and kept at the highest temperature for one hour before being cooled. The de-binder process lasts 18 hour including the cooling time. Alumina of 90% (porosity: 20%) is used as a setter.

### (4) Sintering process

The degreased body is heated from the atmospheric air to the highest temperature and kept at the highest temperature for one hour before being cooled. The sintering time lasts 18 hour including the cooling time. The setter used in the de-binder process is directly used.
The flexural strength of the obtained bone prosthetic agent is 6.1 MPa.

FIG. 14 is a photo, instead of a drawing, showing artificial bone obtained by assembling artificial bone constituent units produced according to Example 1. According to the present invention, as shown in FIG. 14, an artificial bone fitted to the shape of a living body can be custom-made.

### Example 2

Artificial bone constituent units are produced in the same manner as in Example 1 except that the shape of the die is changed.
FIG. 15 shows photos, instead of drawings, showing artificial bone constituent units produced according to Example 2. FIG. 15A shows the artificial bone constituent unit in the shape of a portion of a ring. FIG. 15B shows the artificial bone constituent unit forming a portion of the outer circumference of a ring and the artificial bone constituent unit forming a portion of a ring therewithin. According to the present invention, as shown in FIG. 15, an artificial bone fitted to the shape of a living body can be custom-made.

### Industrial Applicability

The present invention can be used in the field of medical materials.

### Reference Signs List

- 1: Artificial bone constituent unit
- 3: Artificial bone
- 5: First hole
- 7: Body portion
- 9, 11: Connecting portion
- 21: Second hole
- 23: Third hole
- 24, 25, 26: Groove
- 27: Convex portion
- 28: Concave portion
- 29: Concave portion
- 31: Convex portion
- 33, 35: Side face
- 36: Top surface
- 37: Undersurface
- 38: Second hole
- 39: Second hole
- 40: Artificial bone constituent system
- 41: First artificial bone constituent unit group
- 42: First artificial bone constituent unit
- 43: First hole
- 44: Body portion
- 45: Left side face
- 46: Right side face
- 47, 48: Connecting portion
- 49: Front
- 50: Rear face
- 51, 52: Second connecting portion
- 61: Second artificial bone constituent unit group
- 62: Second artificial bone constituent unit
- 63: First hole
- 64: Body portion
- 65: Left side face
- 66: Right side face
- 67, 68: First connecting portion
- 69: Front
- 70: Rear face
- 71, 72: Second connecting portion
- 82: First artificial bone constituent unit
- 84: First hole
- 86: Second hole
- 88: Body portion
- 90: Side face
- 92: Connecting portion
- 94: Front
- 96: Rear face
- 102: Second artificial bone constituent unit
- 104: First hole
- 106: Second hole
- 108: Body portion
- 110: Side face
- 112: Connecting portion
- 114: Front
- 116: Rear face
- 122: Third artificial bone constituent unit
- 124: First hole
- 126: Second hole
- 128: Body portion
- 130: Side face
- 132: Connecting portion
- 139: Front
- 141: Rear face

## Claims

1. An artificial bone constituent unit (1) which is used as an artificial bone (3) by assembling a plurality of artificial bone constituent units into an intended shape;
wherein the artificial bone constituent unit (1) comprise
a body portion (7) including a first hole (5),and
connecting portions (9, 11) mounted on the body portion (7),
wherein the connecting portions (9, 11) are used to connect the artificial bone constituent unit (1) to one or a plurality of other artificial bone constituent units (13, 15),
such that, the artificial bone (3) in a desired shape can be obtained.

2. The artificial bone constituent unit in accordance with claim 1,
wherein the first hole (5) communicates with holes (17, 19) in the other artificial bone constituent units (13, 15) connected to the artificial bone constituent unit (1).

3. The artificial bone constituent unit in accordance with claim 1,
wherein a diameter of the first hole (5) is 0.1 mm or more and 1 cm or less.

4. The artificial bone constituent unit in accordance with claim 1,
wherein the body portion (7) further comprises a second hole (21) and a third hole (23),
the second hole (21) is perpendicular to the first hole (5), the third hole (23) is perpendicular to the first hole (5) and the second hole (21).

5. The artificial bone constituent unit in accordance with claim 1,
wherein the body portion (7) is a block in a hexahedral structure.

6. The artificial bone constituent unit in accordance with claim 1,
wherein the body portion (7) is an isosceles trapezoidal column.

7. The artificial bone constituent unit in accordance with claim 1,
wherein the body portion (7) is a column and cross section of the column is a portion of a ring.

8. The artificial bone constituent unit in accordance with claim 1,
wherein the body portion (7) is a polygonal column.

9. The artificial bone constituent unit in accordance with claim 1,
wherein the body portion 7 is a triangular prism.

10. The artificial bone constituent unit in accordance with claim 1,
wherein the body portion (7) further comprises a first groove 24 on the surface thereof.

11. The artificial bone constituent unit in accordance with claim 1,
wherein the connecting portions (9, 11) further comprise second grooves (25, 26).

12. The artificial bone constituent unit in accordance with claim 1,
wherein the connecting portions (9, 11) are a convex portion (27) or a concave portion (28),
the convex portion (27) or the concave portion (28) of the artificial bone constituent unit (1) fits into a concave portion (29) or a convex portion (31) of the other connected artificial bone constituent units (13, 15) respectively,
such that, the artificial bone constituent unit (1) is connected to the other artificial bone constituent units (13, 15).

13. The artificial bone constituent unit in accordance with claim 1,
wherein the body portion (7) is an isosceles trapezoidal column,
the connecting portions (9, 11) at least provided on isosceles side faces (33, 35) of the body portion (7)
because of the above configuration, the artificial bone constituent unit (1) is connected to one or a plurality of the other artificial bone constituent units (13, 15) via the isosceles side faces.

14. An artificial bone constituent system (40) including a plurality of artificial bone constituent units,
wherein the artificial bone constituent system (40) comprises
at least a first artificial bone constituent unit group (41) and a second artificial bone constituent unit group (61),
The first artificial bone constituent unit group (41) and the second artificial bone constituent unit group (61) have a plurality of first artificial bone constituent units (42) and a plurality of second artificial bone constituent units (62) respectively,
the first artificial bone constituent unit (42) has
a body portion (44) that has a first hole (43) and a hexahedral structure, first connecting portions (47, 48) mounted on a left side face (45) and a right side face (46) of the body portion (44), and
second connecting portions (51, 52) mounted on a front (49) and a rear face (50) of the body portion (44),
the first connecting portions (47, 48) are used to connect the first artificial bone constituent unit (42) to one or two artificial bone constituent units (53, 54) contained in the first artificial bone constituent unit group (41)
the second artificial bone constituent unit (62) has
a body portion (64), that has a first hole (63) and a hexahedral structure,
first connecting portions (67, 68) mounted on a left side face (65) and a right side face (66) of the body portion (64), and
second connecting portions (71, 72) mounted on a front (69) and a rear face (70) of the body portion (64),
the first connecting portions (67, 68) are used to connect the second artificial bone constituent unit (629 to one or two artificial bone constituent units (73, 74) contained in the second artificial bone constituent unit group (61)
the second connecting portion (51) of the first artificial bone constituent unit (41) is connected to the second connecting portion (72) of the second artificial bone constituent unit (62), accordingly, the first artificial bone constituent unit (42) and the second artificial bone constituent unit (62) are connected.

15. The artificial bone constituent system in accordance with claim 14
if one or two first artificial bone constituent units are connected to the first artificial bone constituent unit group (41) via one of the first connecting portions (47, 48) of the first artificial bone constituent unit 42, the shape of a ring or a portion thereof is formed,
if one or two second artificial bone constituent units are connected to the second artificial bone constituent unit group (61) via one of the first connecting portions (67, 68) of the second artificial bone constituent unit (62), the shape of a ring or a portion thereof is formed,
the shape of the body portion (44) of the first artificial bone constituent unit (42) and the body portion (64) of the second artificial bone constituent unit (62) is a shape in which the shape of a ring or a portion thereof by the first artificial bone constituent unit group (41) is positioned on an outer circumference of the shape of a ring or a portion thereof by the second artificial bone constituent unit group (61).

16. An artificial bone constituent system (80) having a plurality of artificial bone constituent units
wherein the artificial bone constituent system (80) comprises
a plurality of first artificial bone constituent units (82) and a plurality of second artificial bone constituent units (102)
the first artificial bone constituent unit (82) has
a body portion (88) that has a first hole (84) and a second hole (86) and has the shape of a regular triangular prism, and
a connecting portion (92a∼92c) mounted on a side face (90a∼90c) of the body portion (88),
the connecting portion (92a∼92c) is used to connect to another first artificial bone constituent unit or the second artificial bone constituent unit (102),
the second artificial bone constituent unit (102) has
a body portion (108) that has a first hole (104) and a second hole (106) and has the shape of a regular triangular prism, and
a connecting portion (112a∼112c) mounted on a side face (110a∼110c) of the body portion (108),
the connecting portion (112a∼112c) is used to connect to the first artificial bone constituent unit (82) or another second artificial bone constituent unit.

17. The artificial bone constituent system in accordance with claim 16
wherein the artificial bone constituent system further comprises
a third artificial bone constituent unit (122),
the a third artificial bone constituent unit (122) has
a body portion (128) that has a first hole (124) and a second hole (126) and has the shape of a regular hexagonal prism, and
a connecting portion (130a∼130f) mounted on a side face (130a∼130f) of the body portion (128),
the connecting portion (132a∼132f) is used to connect to the first artificial bone constituent unit (82), the second artificial bone constituent unit (122), or other third artificial bone constituent unit.
